Europäisches Patentamt

European Patent Office

Office européen des brevets

(19)

(11) Numéro de publication : **0 020 278**
**B1**

(12) # FASCICULE DE BREVET EUROPÉEN

(45) Date de publication du fascicule du brevet :
22.06.83

(51) Int. Cl.³ : **C 12 Q 1/34, G 01 N 33/50**

(21) Numéro de dépôt : 80400779.7

(22) Date de dépôt : 02.06.80

(54) **Procédé de diagnostic in vitro de la fibrose kystique.**

(30) Priorité : 01.06.79 FR 7914234

(43) Date de publication de la demande :
10.12.80 Bulletin 80/25

(45) Mention de la délivrance du brevet :
22.06.83 Bulletin 83/25

(84) Etats contractants désignés :
BE CH DE GB IT LI LU NL

(56) Documents cités :
US A 3 902 847
CHEMICAL ABSTRACTS, vol. 88, no. 4, 23 janvier
1978, page 171, abrégé no. 33602t COLUMBUS,
Ohio (US) S. HIRANI et al. : « Measurement of the
alpha-mannosidase activities in human plasma
by a differential assay »
CHEMICAL ABSTRACTS, vol. 82, no. 7, 17 février
1975, page 326, abrégé no. 54735e COLUMBUS,
Ohio (US) J. BUTTERWORTH et al. : « Lysosomal
enzyme levels In human amniotic fluid cells in
tissue culture. III. beta-Glucuronidase, N-acetyl-
beta-D-glucosaminidase, alpha-mannosidase,
and acid phosphatase »
CHEMICAL ABSTRACTS, vol. 88, no. 7, 13 février
1978, page 255, abrégé no. 60814v COLUMBUS,
Ohio (US) O. HOSLI et al. : « Cystic fibrosis :
leakage of lysosomal enzymes and of alkaline
phosphatase into the extracellular space »

(73) Titulaire : **INSTITUT PASTEUR**
**25-28, rue du Docteur Roux**
**F-75724 Paris Cedex 15 (FR)**

(72) Inventeur : **Hösli, Peter**
**159, rue Blomet**
**F-75015 Paris (FR)**

(74) Mandataire : **Gutmann, Ernest et al**
**Cabinet Plasseraud 84, rue d'Amsterdam**
**F-75009 Paris (FR)**

# 0 020 278

## Procédé de diagnostic *in vitro* de la fibrose kystique

La fibrose cystique ou kystique (visée ci-après par les initiales « CF »), également dénommée mucoviscidose, est une maladie génétique récessive autosomale extrêmement grave, particulièrement fréquente dans les populations d'origine européenne. En France un nouveau-né sur 2 000 à 2 500 en est affecté. Cette maladie se manifeste en général aussitôt après la naissance. Les syndromes les plus caractéristiques consistent dans le développement d'obstructions pulmonaires chroniques, d'insuffisances pancréatiques, un défaut de métabolisation des graisses et une sensibilité extrême aux infections, notamment au niveau des régions respiratoires. La mort s'ensuit habituellement au cours des toutes premières années de la vie de l'enfant.

La mucoviscidose est une affection familiale se transmettant selon le mode autosomal récessif. Il semble à ce jour établi que sont susceptibles d'être affectés de cette maladie les enfants dont le patrimoine génétique résulte de la recombinaison chromosomique au moment de la fécondation des patrimoines génétiques du père et de la mère, lorsque tous deux sont porteurs d'une mutation chromosomique spécifique, sans pour autant être eux-mêmes affectés de cette maladie. Ces parents seront ci-après dits «CF-hétérozygotes». Bien entendu, cette expression s'étend également à leurs cellules et, d'une façon générale, à leur patrimoine génétique propre. Sont donc susceptibles d'être affectés de la maladie les enfants qui reçoivent en partage ces défauts génétiques de leurs deux parents, eux-mêmes étant dits par la suite «CF-homozygotes ».

Cette anomalie génétique devrait donc pouvoir être dépistée aussitôt que possible, les chances de survie du nouveau-né potentiellement affecté de la maladie étant d'autant meilleures qu'il peut sans attendre faire l'objet d'une thérapie intensive. Il serait mieux encore de dépister cette anomalie dans la phase néonatale, éventuellement même dans la toute première phase de la grossesse, à un moment où une interruption de grossesse thérapeutique pourrait éventuellement encore être indiquée.

Certes, il existe déjà à ce jour des techniques permettant d'apprécier sur des prélèvements de liquide amniotique des anomalies chromosomiques, lorsqu'elles se manifestent par des modifications d'ordre structurel. Ces techniques s'avèrent cependant beaucoup plus difficiles à ce jour lorsque ces anomalies sont invisibles à l'œil et sont l'expression d'erreurs futures de contrôle ou de régulation du métabolisme cellulaire.

En effet, selon des hypothèses qui trouvent un soutien expérimental de plus en plus solide, le défaut génétique qui est à l'origine de la fibrose cystique paraît affecter de nombreuses enzymes qui jouent un rôle dans la digestion intra-cellulaire. En particulier, ce défaut génétique paraît affecter entre autres le système lysosomial, cette affection se manifestant par des libérations d'hydrolases dans des espaces extracellulaires, résultant par conséquent en des déficiences intracellulaires multiples en ces hydrolases.

L'hypothèse a été formulée, en ce qui concerne plus particulièrement les hydrolases lysosomiales, que le défaut génétique du genre en question affecte certains marqueurs portés par ces hydrolases, marqueurs qui servent à ancrer ces hydrolases sur des récepteurs de membranes lysosomiaux spécifiques. Ces marqueurs paraissent devoir être constitués par des oligosaccharides phosphorylés fixés sur les protéines enzymatiques à l'occasion d'un processus secondaire intervenant lors de leur passage à travers les cavités du réticulum endoplasmique.

Les procédés de diagnostic connus à ce jour sont difficiles à mettre en œuvre et non appropriés à des dépistages systématiques de l'anomalie génétique du genre en question. Le procédé le plus répandu est un test dit « test de sueur », lequel vise à déterminer les taux anormalement élevés en sodium et en chlore que contient la sueur des enfants affectés par la fibrose cystique ou kystique. Il est d'une mise en œuvre délicate, requérant la présence du patient au laboratoire. Il ne peut être effectué avec un niveau raisonnable de certitude quant aux résultats que par des techniciens très expérimentés. Il est naturellement inapproprié à la réalisation de dépistages néonataux. Les procédés proposés jusqu'à ce jour pour les dépistages néonataux n'ont guère été développés, tant en raison des résultats ambigus auxquels ils conduisent que de la difficulté technique de leur mise en œuvre.

L'invention a pour but de remédier à ces inconvénients, plus particulièrement de fournir un procédé permettant un diagnostic à la fois plus aisé et plus sûr que les méthodes connues à ce jour, qui puisse être mis en œuvre par des personnes n'ayant pas le niveau de compétence technique exigé des techniciens mettant en œuvre le «test de sueur ».

Le procédé selon l'invention a encore pour but la réalisation du test de dépistage, non seulement chez les nouveau-nés ou d'une façon plus générale les enfants, mais aussi chez l'embryon et de préférence même dans les toutes premières semaines de la grossesse.

L'invention vise encore à l'obtention d'un procédé permettant le dépistage systématique au niveau des populations de ceux des individus éventuellement porteurs du défaut génétique « CF-hétérozygoté ». Un tel test de diagnostic systématique pourrait, par exemple, être mis en œuvre à l'occasion des visites médicales prénuptiales de plus en plus couramment prescrites par les législations nationales.

L'invention est basée sur une approche différentielle des comportements de l'une au moins des enzymes déterminées qui sont susceptibles à la fois d'avoir un rôle dans la digestion intracellulaire et d'être au moins partiellement affectées du fait d'une éventuelle atteinte génétique de l'individu dont elle provient, que celui-ci soit « CF-homozygote » ou même seulement « CF-hétérozygote ». Par exemple, il a

2

été constaté que l'enzyme provenant d'un individu « CF-homozygote » ou même «CF-hétérozygote » présentait une thermolabilité plus importante que la même enzyme provenant d'un individu témoin chromosomiquement dépourvu de tout défaut génétique du type de ceux tenus pour responsables de la mucoviscidose.

L'invention est basée sur la recherche, pour chaque enzyme susceptible d'être prise en considération, des conditions limites de température dans lesquelles l'enzyme provenant d'un individu « CF-homozygote » et/ou « CF-hétérozygote » est susceptible de subir une inactivation ou une dégradation plus importante que la même enzyme provenant d'un individu non porteur de ce défaut génétique. L'invention s'appuie en effet sur la démonstration ici rapportée que de telles conditions limites de température peuvent exister, particulièrement que ces conditions limites peuvent même être déterminées, dans lesquelles des enzymes déterminées provenant d'un témoin normal restent stables, alors que les mêmes enzymes, placées dans les mêmes conditions, sont susceptibles de subir une dégradation plus ou moins prononcée, selon qu'elles sont originaires d'un individu « CF-homozygote » ou « CF-hétérozygote ».

Le procédé selon l'invention est donc caractérisé en ce que, partant d'un milieu biologique provenant soit directement de l'individu chez lequel est recherchée l'affection ou même la potentialité de transmettre l'affection, soit d'un milieu de culture ou de suspension de cellules d'une culture extracorporelle de cellules préalablement prélevées sur cet individu, on détermine les conditions limites de température dans lesquelles au moins l'une des enzymes intervenant au niveau de la digestion intracellulaire, plus particulièrement des hydrolases du type de celles qui sont susceptibles d'être affectées par le ou les défauts génétiques propres aux individus « CF-hétérozygotes » ou « CF-homozygotes » peut, lorsqu'elle est originaire d'un individu normal, rester stable, voire ne subir qu'une cinétique d'inactivation ralentie, et au contraire, subir une cinétique d'activation accélérée, voire une inactivation totale lorsqu'elle est originaire d'un individu « CF-hétérozygote » ou « CF-homozygote » et que l'on repère ensuite qualitativement ou quantitativement sur chacun des échantillons de milieu biologique à tester placés dans les mêmes conditions de température, les cinétiques d'inactivation accélérées ou altérations plus rapides éventuellement observables de la même enzyme, qui peuvent alors être corrélées au caractère « CF-hétérozygote » ou « CF-homozygote » des cellules correspondantes.

Il est entendu que lorsque l'on parle dans ce qui suit « d'individus », il s'agit aussi bien d'enfants que de personnes adultes (en ce qui concerne plus particulièrement les « CF-hétérozygotes ») et d'embryons, ne fût-ce qu'au premier stade de la grossesse.

La susdite détermination des conditions limites de température implique donc pour chaque enzyme étudiée la recherche de la température, plus particulièrement l'intervalle de températures dans lequel ces comportements différentiels peuvent être observés.

Bien entendu les susdites conditions limites de température en rapport avec toute enzyme déterminée peuvent être déterminées une fois pour toutes. En particulier il sera donc désirable de déterminer des seuils de variations des cinétiques différentielles d'inactivation ou des degrés d'altération de l'enzyme considérée, lorsque celle-ci est placée dans les susdites conditions limites de température, qui permettront au technicien, d'après les résultats des mesures relatives à ces variations de cinétique ou de degré d'altération effectuées sur un échantillon testé, de rapidement déterminer si celui-ci provenait d'un individu vraisemblablement sain, « CF-hétérozygote » ou « CF-homozygote ».

En particulier lorsque les paramètres pris en considération consistent en des cinétiques d'inactivation différentielles, les susdits seuils peuvent être constitués par les rapports déterminés des pourcentages d'inactivation de l'enzyme déterminée obtenus dans les susdites conditions de température dans lesquelles les comportements différentiels sont susceptibles de se manifester, aux pourcentages d'inactivation observables sur des enzymes provenant d'individus sains, ces enzymes ayant été placées dans les mêmes conditions de température que les enzymes étudiées.

Lorsque ce rapport reste élevé, proche de 1, (la cinétique d'inactivation de l'enzyme étudiée étant alors proche de celle provenant d'individus sains) il pourra être conclu avec un haut niveau de probabilité que les cellules dont provenait l'enzyme étudiée appartenaient à un individu sain. Lorsque ce rapport tombera au-dessous d'une première valeur-seuil, nettement inférieure à 1, mais restant néanmoins supérieure à une seconde valeur-seuil encore plus faible, il pourra être conclu que les cellules dont provenait l'enzyme appartenaient vraisemblablement à un individu « CF-hétérozygote ». Lorsque ce rapport tombera sous la seconde valeur-seuil susdite, il pourra être conclu que vraisemblablement l'enzyme provenait de cellules appartenant à un individu « CF-homozygote ».

Bien entendu, l'on pourra également parler en pourcentage d'inactivation vis-à-vis d'une valeur absolue, comme il sera indiqué plus loin à propos de l'étude des cinétiques d'inactivation de l'$\alpha$-mannosidase, après traitement du milieu contenant cette enzyme pendant une durée déterminée, à une température sous laquelle le susdit comportement différentiel est susceptible de se manifester, par exemple 41 °C ou 40,5 °C lorsqu'il s'agit de l'$\alpha$-mannosidase.

D'une façon générale le procédé selon l'invention peut encore être caractérisé en ce que, partant d'un milieu tel qu'il a été défini plus haut et antérieurement en contact avec les cellules sur lesquelles doit être effectué le diagnostic in vitro, on place ledit milieu dans les conditions de température préalablement déterminées permettant à l'une au moins de celles des enzymes qui ont été libérées dans le milieu et qui sont susceptibles de porter une modification lorsqu'elles sont originaires de cellules affectées par un défaut génétique corrélable à la fibrose kystique, de manifester une cinétique d'inactivation différentielle vis-à-vis de la cinétique d'inactivation de la même enzyme provenant de cellules saines dans les mêmes conditions prédéterminées, voire même une inactivation quasi-totale, et on repère les variations éventuelles vis-à-vis de la normale de ladite cinétique d'inactivation, pour les corréler selon la variation mesurée, à l'état soit normal, soit « CF-hétérozygote », soit encore « CF-homozygote » des cellules dont l'enzyme était originaire. Ledit repérage peut notamment consister en la mesure, au bout dun temps également prédéterminé, du degré d'inactivation éventuellement atteint, ce degré étant alors corrélable à l'un des états susmentionnés desdites cellules.

Dans des modes de mise en œuvre préférés de l'invention, on met à profit les différences de comportement thermique des enzymes telles que l'α-mannosidase ou la phosphatase-acide, dont il a été constaté qu'à une température supérieure à 40 °C, notamment comprise entre 40 °C et 42 °C pour la première et supérieure à 36 °C, notamment comprise entre 36 et 38 °C pour la seconde, les enzymes correspondantes pouvaient subir une inactivation plus rapide lorsqu'elles provenaient d'individus « CF-homozygotes » plutôt que d'individus « CF-hétérozygotes » ou encore lorsqu'elles provenaient d'individus « CF-hétérozygotes » plutôt que d'individus normaux.

Ces enzymes ne sont que des exemples d'hydrolases dont la nature paraît être altérée par l'existence de défauts génétiques chromosomiques chez les individus « CF-homozygotes » ou « CF-hétérozygotes ». De tels phénomènes doivent cependant pouvoir être constatés également pour d'autres hydrolases, notamment des hydrolases lysosomiales, telles que l'α-glucosidase ou l'α-fucosidase.

Dans les modes de réalisation préférés de l'invention mentionnés plus haut, l'on soumet par conséquent les échantillons biologiques d'étude distincts et contenant les enzymes en question libérées par les cellules des individus à l'étude auparavant à leur contact, à des traitements thermiques pendant des intervalles de temps respectivement croissants à une température contenue dans l'intervalle où le susdit comportement différentiel est éventuellement susceptible de se manifester, de façon à provoquer l'apparition d'une cinétique d'inactivation plus rapide de l'enzyme pour le cas où l'échantillon traité proviendrait d'un individu « CF-homozygote » ou «CF-hétérozygote ».

Dans le cas de l'α-mannosidase, la température de 41 °C est préférée. Dans le cas de la phosphatase-acide la température de 36,5 °C est préférée.

Un milieu biologique préféré est constitué par un plasma sanguin. Le fibrinogène ou les facteurs susceptibles d'intervenir de façon essentielle dans la coagulation, peuvent être retirés ou ils doivent être totalement inhibés.

Le déclenchement de phénomènes de coagulation aurait pour effet la libération par les thrombocytes d'hydrolases thermostables dont la présence dans les échantillons étudiés fausserait totalement la cinétique de « thermoinactivation » des enzymes thermolabiles du genre de celles dont le comportement doit être étudié dans les tests de diagnostic *in vitro* conformément à l'invention.

L'inhibition des facteurs de coagulation peut être réalisée à l'aide de tout anti-coagulant approprié, par exemple l'héparine.

Tout autre milieu biologique peut être envisagé dans la mesure où il aura pu se trouver en contact avec des cellules aptes à libérer dans ce milieu les enzymes étudiées. Il s'agit éventuellement d'autres liquides biologiques humains, par exemple l'urine. Un autre milieu biologique peut être constitué par le liquide amniotique prélevé chez la femme enceinte, liquide pouvant lui-même alors contenir des cellules aptes à libérer dans ce milieu les enzymes du genre en question. Ces milieux biologiques peuvent également être constitués par les milieux de culture classiques appropriés à la culture de fibroblaste humain, comme il est bien connu des biologistes.

De préférence, les opérations dont question ci-dessus sont effectuées sur ces milieux maintenus à pH sensiblement neutre. D'une façon générale on peut opérer à pH 5-9.

De préférence encore, ces essais sont réalisés sur des quantités extrêmement faibles de liquide pour éviter une surabondance d'enzymes dans l'échantillon étudié et par conséquent un éventuel masquage des phénomènes observés.

Avantageusement, les échantillons du liquide biologique traité ont un volume compris entre 0,1 et 100 µl.

Une condition importante à la bonne marche du procédé selon l'invention, surtout lorsqu'elle met en œuvre des cinétiques comparées d'inactivation des enzymes, réside dans la protection des échantillons traités thermiquement contre l'évaporation.

Avantageusement, les réactions sont effectuées en tubes, la protection contre l'évaporation étant alors obtenue au moyen d'une couche d'huile de densité inférieure à 1, recouvrant les volumes liquides traités, n'ayant qu'une tension de vapeur extrêmement réduite, sinon pratiquement nulle à la température à laquelle sont effectués les essais d'inactivation tels que définis plus haut. Une telle huile est par exemple constituée par une huile de paraffine.

On peut avoir recours à toute technique de dosage quantitative de l'enzyme étudiée après les traitements thermiques qui ont été décrits.

Avantageusement encore, on aura recours de façon en soi connue aux techniques mettant en jeu l'action de l'enzyme sur l'un de ses substrats, notamment du type de ceux dont la transformation peut être suivie par des méthodes photocolorimétriques. Dans de tels cas, l'huile utilisée devra être choisie non fluorescente dans les conditions expérimentales envisagées. L'huile de paraffine non fluorescente UVASOL fabriquée par la société MERCK de Darmstadt (R.F.A.) est appropriée dans les modes de mise en œuvre préférés décrits ci-après du procédé selon l'invention.

Les mesures dont font état les exemples ont été faites avec un spectrophotofluorimètre PERKIN-ELMER MPF-4.

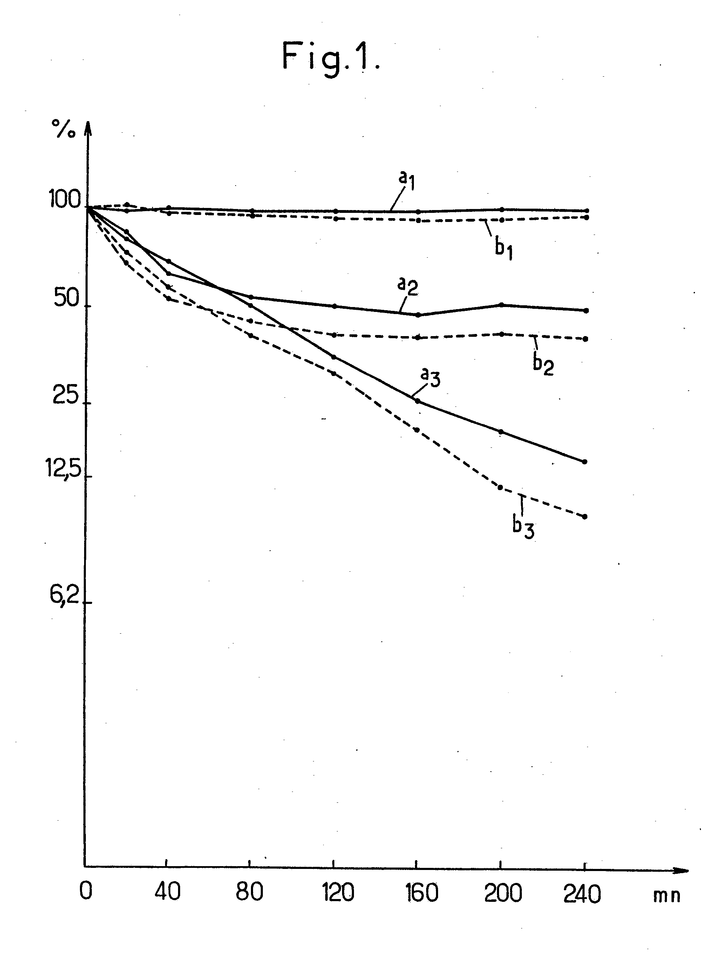

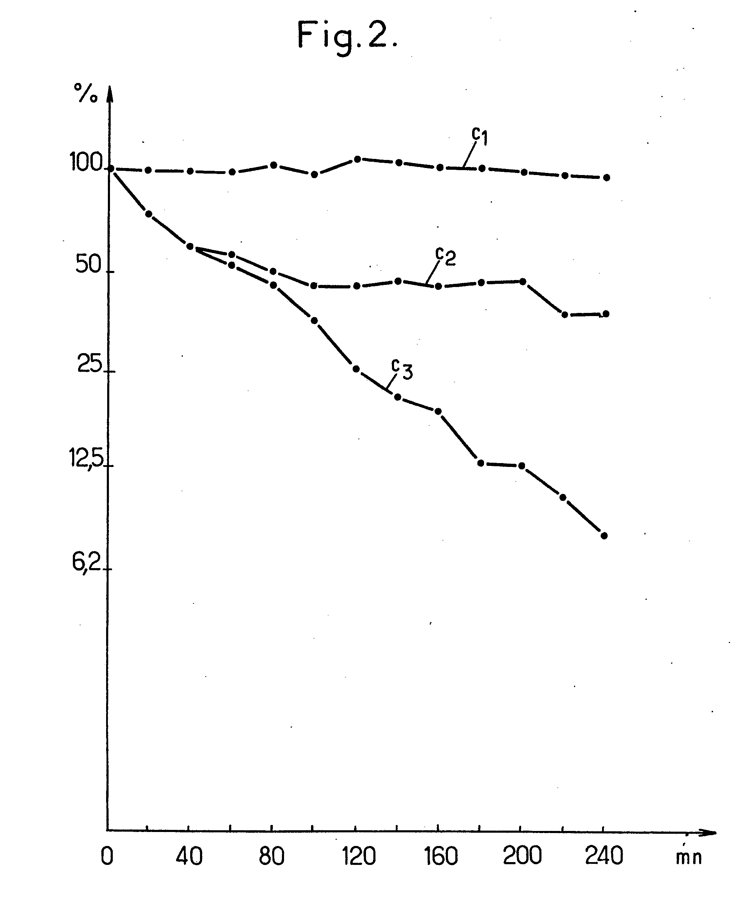

D'autres caractéristiques de l'invention apparaîtront encore au cours de la description des modes de mise en œuvre de celle-ci dans les exemples qui suivent. Référence sera faite dans ces exemples au dessin dans lequel : la fig. 1 est représentative des taux d'inactivation (exprimés en % sur l'axe des ordonnées) des enzymes étudiées dans des échantillons de plasma sanguin, tels que définis ci-dessus, en fonction de durées croissantes d'inactivation thermique (exprimée en minutes sur l'axe des abscisses), ces enzymes provenant, respectivement, de sujets normaux, « CF-homozygotes » et « CF-hétérozygotes » ; la fig. 2 représente les variations des mêmes phénomènes dans les mêmes conditions à l'égard d'enzymes contenues dans les surnageants des milieux dans lesquels des cellules provenant respectivement d'individus normaux, « CT-homozygotes » et « CF-hétérozygotes », avaient été cultivées.

## Exemple I

Du sang veineux est recueilli dans des tubes en matière plastique contenant 25 unités internationales d'héparine pour chaque millilitre de sang. Ces tubes sont alors placés dans de l'eau glacée et doivent être traités dans l'heure qui suit. Pour précéder à une séparation des cellules sanguines et du plasma, on soumet les tubes à une centrifugation séquentielle dans une centrifugeuse réfrigérée à 4 °C d'abord, pendant 5 minutes à 400 g et ensuite pendant 10 minutes à 3 000 g. Des parties aliquotes de plasma sont ensuite réparties dans des tubes d'Eppendorf, congelées et maintenues dans de l'azote liquide jusqu'à l'usage postérieur. Ces parties aliquotes, après décongélation dans l'air ambiant, doivent être immédiatement traitées et ne doivent pas être recongelées.

Les étapes préférées du traitement sont alors réalisées dans l'ordre suivant :

1. Les échantillons de plasma décongelés sont dilués au dixième avec une solution de chlorure de sodium à 0,9 % et gardés dans l'eau glacée.

2. Des échantillons de 10 microlitres du plasma ainsi dilué sont répartis dans des tubes d'Eppendorf.

3. 25 microlitres d'huile UVASOL sont ajoutés à chacun des tubes, ceux-ci étant ensuite centrifugés à 6 000 g pendant 20 secondes. Cette opération vise à former une couche d'huile protégeant l'échantillon de plasma contre l'évaporation, à l'occasion du traitement d'inactivation thermique ultérieur.

4. On traite les divers échantillons au bain-marie à 41 °C, lorsque l'enzyme étudiée est l'α-mannosidase, et à 36,5 °C, lorsque l'enzyme étudiée est la phosphatase-acide, pendant des intervalles de temps respectivement croissants de 0 à 240 minutes. Les instants auxquels sont démarrés ces traitements d'inactivation thermique sur les divers tubes sont décalés dans le temps, de façon à ce que les durées voulues des traitements thermiques dans les tubes respectifs viennent à échéance au même instant.

Les inactivations thermiques en cours sur les tubes étudiés sont interrompues simultanément par mise de tous les tubes dans un bain d'eau glacée pendant 5 minutes.

5. 10 microlitres d'une solution d'un substrat au sein d'un tampon sont ajoutés à chacun des tubes, ceux-ci étant alors recentrifugés à 6 000 g pendant 20 secondes, afin de s'assurer du passage certain du substrat au travers de la couche d'huile et sa mise en contact avec la solution de plasma étudié.

La solution de substrat utilisée est une solution de 10 mM de 4-méthylumbelliperyl-α-D-mannopyranoside (ou de 4-Mu-phosphate pour la phosphatase-acide) dans un tampon citrate 0,1 M. Le pH final du milieu est 5,4.

6. Les divers tubes sont réincubés à 30 °C pendant 90 minutes au bain-marie.

7. Les incubations de tous ces échantillons sont interrompues à nouveau simultanément par mise des tubes correspondants dans un bain d'eau glacée pendant 5 minutes.

8. On dilue alors tous les échantillons avec chaque fois 1 millilitre de la solution tampon de carbonate pH 10,7 et, après recentrifugation pendant 20 secondes à 6 000 g, on effectue une mesure colorimétrique dans des cuvettes conventionnelles de quartz au spectrophotofluorimètre (longueur d'onde d'excitation 360 mμ ; longueur d'onde d'émission 448).

Les résultats observés feront l'objet d'un commentaire après l'exposé des conditions opératoires de l'exemple II, cette fois-ci appliqué à des cultures de cellules provenant de biopsies de peau chez des témoins normaux, des malades « CF-homozygotes » et des porteurs « CF-hétérozygotes ».

## Exemple II

Les cultures de cellules de fiboroblaste, prélevées par des techniques classiques, sont placées dans un milieu HAM F10 (GIBCO), auquel a été ajouté du FCS (GIBCO), à raison de 15 %. Les cultures sont maintenues pendant trois jours à l'état confluent à 37 °C dans le milieu de culture. Après lavage des cellules, celles-ci sont remises en suspension et maintenues pendant deux jours dans un milieu ci-après dit « milieu de récupération », constitué pour chacune des cultures par 2,2 millilitres de HAM F10, auquel avait été ajouté 5 % de FCS, lequel avait été antérieurement inactivé thermiquement pendant trois jours à 56 °C. Les cellules sont ensuite séparées par centrifugation du « milieu de récupération » pendant 2 minutes à 5 000 g et le « surnageant de récupération » est récupéré. Des parties aliquotes de ce milieu

# 0 020 278

de récupération sont réparties dans des tubes d'Eppendorf, congelées et maintenues dans de l'azote liquide jusqu'à la réalisation des opérations ultérieures de dosage.

La procédure appliquée à l'occasion de ces dosages est ensuite celle qui a été décrite à l'exemple I (étapes 1 à 8), sauf que la durée d'incubation à l'étape 6°) est portée à 180 minutes.

La fig. 1 est représentative des résultats qui ont été obtenus, d'une part, par l'étude des courbes d'inactivation thermique de l'$\alpha$-mannosidase (courbes $a_1$, $a_2$, $a_3$) et de la phosphatase-acide (courbes $b_1$, $b_2$, $b_3$) sur des échantillons de plasma traités dans les conditions qui ont été indiquées ci-dessus dans l'exemple I.

Les courbes $a_1$, $b_1$ ont été effectuées sur un échantillon sanguin appartenant à un témoin normal, les courbes $a_2$, $b_2$ résultent des essais effectués sur le plasma originaire d'un individu « CF-hétérozygote obligatoire » (ascendant direct d'un enfant atteint de mucoviscidose).

Enfin, les courbes $a_3$, $b_3$ expriment les résultats obtenus sur un échantillon de plasma provenant d'un sujet « CF-homozygote ».

Les courbes de la fig. 1 montrent que, dans les conditions opératoires utilisées, les enzymes $\alpha$-mannosidase et phosphatase-acide ne subissent pratiquement aucune inactivation.

On observe au contraire, dans le cas d'individu « CF-hétérozygotes » (courbes $a_2$, $b_2$) une inactivation partielle, pendant les 80 premières minutes, de ceux des échantillons exposés au traitement thermique d'inactivation pendant les durées correspondantes, cette inactivation n'étant cependant que partielle, comme il résulte de l'aplatissement au niveau approximativement 50 % des courbes $a_2$, $b_2$ dans les échantillons soumis au traitement d'inactivation thermique pendant des durées plus importantes (80 à 240 minutes).

Par contre, on constate chez les individus « CF-homozygotes » une inactivation croissante dans le temps, tendant vers zéro au fur et à mesure qu'ont crû les temps d'inactivation auxquels ont été soumis les échantillons correspondants.

La fig. 2 est représentative des résultats obtenus à partir des « surnageants de récupération » provenant des cultures cellulaires provenant des mêmes individus respectivement témoins (courbes $c_1$), « CF-hétérozygotes obligés » (courbe $c_2$) et « CF-homozygotes » (courbe $c_3$).

Le comportement de l'enzyme étudiée dans l'exemple II ($\alpha$-mannosidase) est tout à fait semblable à celui remarqué à propos des $\alpha$-mannosidases des échantillons de plasma sanguin de l'exemple I.

Les tableaux 1a, 1b et 1c qui suivent donnent les résultats qui ont été obtenus sur des séries plus importantes d'individus appartenant aux trois catégories déjà mentionnées plus haut.

Les nombres figurant dans ces différents tableaux correspondent aux pourcentages d'activité résiduelle $\alpha$-mannosidase/phosphatase-acide qui subsistent après des durées de traitement d'inactivation thermique dans les conditions indiquées dans l'exemple I sur des échantillons de plasma provenant des différents individus donneurs et maintenus aux températures respectives de 41 °C et 36,5 °C pendant 40 minutes, 120 minutes et 200 minutes respectivement, vis-à-vis des activités mesurées de ces enzymes dans des échantillons n'ayant pas subi les inactivations thermiques décrites mais ayant été pendant les durées correspondantes maintenus dans des bains de glace.

Les résultats du tableau 1a sont les résultats qui ont été obtenus sur des plasmas provenant de 20 individus normaux. De même dans le tableau 1b figurent les résultats obtenus sur des plasmas sanguins provenant de 22 individus « CF-hétérozygotes obligatoires ». Enfin les résultats figurant au tableau 1c sont ceux qui ont été obtenus sur des plasmas sanguins provenant de 5 individus « CF-homozygotes ».

Figurent également dans ces tableaux les résultats moyens observés ainsi que les déviations standard D.S.

Les résultats figurant dans les tableaux 1a, 1b, 1c témoignent d'une reproductibilité remarquable des résultats observés chez les différents individus d'un même groupe. Dans la mesure où le test selon l'invention met par conséquent en jeu l'étude des inactivations thermiques relatives de l'$\alpha$-mannosidase dans les conditions décrites à l'exemple I, il peut être raisonnablement inféré des résultats qui seront obtenus sur le plasma sanguin de tout individu soumis à l'épreuve de diagnostic selon l'invention, que le pourcentage d'inactivation dans une proportion dépassant 75-80 % au bout de 200 minutes du traitement d'inactivation thermique à 41 °C révèle des « CF-homozygotes » quasi certains.

Lorsque le pourcentage observé de l'inactivation est de l'ordre de 40 à 60 % au bout de 200 minutes, dans les conditions expérimentales ci-dessus décrites, il peut être estimé que les prélèvements sanguins en question proviennent d'individus « CF-hétérozygotes ». Une inactivation de l'ordre de 25 à 35 % témoigne d'une vraisemblance, sinon d'une certitude de l'existence d'un caractère « CF-hétérozygote ».

(Voir Tableaux page 7)

6

## TABLEAU 1a

### INACTIVATION THERMIQUE α-MANNOSIDASE/PHOSPHATASE-ACIDE
(en %)

| témoins normaux | 0' | 40' | 120' | 200' |
|---|---|---|---|---|
| 1 | 100/100 | 99/97 | 99/96 | 99/94 |
| 2 | 100/100 | 97/99 | 98/99 | 99/93 |
| 3 | 100/100 | 97/96 | 96/99 | 87/93 |
| 4 | 100/100 | 99/98 | 97/92 | 97/91 |
| 5 | 100/100 | 84/94 | 85/94 | 87/91 |
| 6 | 100/100 | 95/95 | 96/93 | 95/92 |
| 7 | 100/100 | 95/99 | 94/95 | 94/94 |
| 8 | 100/100 | 97/96 | 98/95 | 96/93 |
| 9 | 100/100 | 99/104 | 97/101 | 96/92 |
| 10 | 100/100 | 97/100 | 94/91 | 100/94 |
| 11 | 100/100 | 98/101 | 100/102 | 100/91 |
| 12 | 100/100 | 103/93 | 104/87 | 103/94 |
| 13 | 100/100 | 106/103 | 95/98 | 100/98 |
| 14 | 100/100 | 98/102 | 94/99 | 91/97 |
| 15 | 100/100 | 97/101 | 94/101 | 92/100 |
| 16 | 100/100 | 97/105 | 95/99 | 95/105 |
| 17 | 100/100 | 99/94 | 102/91 | 100/90 |
| 18 | 100/100 | 99/98 | 93/99 | 86/97 |
| 19 | 100/100 | 99/100 | 94/98 | 92/88 |
| 20 | 100/100 | 98/97 | 94/98 | 84/97 |
| moyenne | 100/100 | 97.7/98.6 | 96/96 | 94.7/94.2 |
| ± D.S. | | ±4.0/±3.4 | ±3.9/±4.0 | ±5.4/±3.9 |

## TABLEAU 1b

### INACTIVATION THERMIQUE α-MANNOSIDASE/PHOSPHATASE ACIDE
(en %)

| CF-hétérozygotes | 0' | 40' | 120' | 200' |
|---|---|---|---|---|
| 1 | 100/100 | 66/71 | 45/45 | 46/48 |
| 2 | 100/100 | 64/56 | 55/47 | 51/50 |
| 3 | 100/100 | 63/56 | 50/41 | 50/41 |
| 4 | 100/100 | 67/69 | 56/50 | 55/48 |
| 5 | 100/100 | 66/58 | 58/52 | 54/49 |
| 6 | 100/100 | 68/53 | 58/42 | 53/39 |
| 7 | 100/100 | 80/69 | 53/50 | 55/45 |
| 8 | 100/100 | 72/68 | 49/49 | 44/45 |
| 9 | 100/100 | 75/79 | 55/56 | 51/46 |
| 10 | 100/100 | 73/76 | 47/53 | 50/53 |
| 11 | 100/100 | 86/77 | 53/41 | 51/41 |
| 12 | 100/100 | 84/70 | 53/40 | 50/44 |
| 13 | 100/100 | 79/77 | 57/49 | 50/48 |
| 14 | 100/100 | 75/76 | 49/46 | 50/49 |
| 15 | 100/100 | 79/70 | 50/39 | 50/40 |
| 16 | 100/100 | 79/71 | 49/45 | 45/41 |
| 17 | 100/100 | 81/68 | 55/47 | 53/48 |
| 18 | 100/100 | 74/72 | 56/37 | 52/28 |

## 0 020 278

### TABLEAU 1b (suite)

### INACTIVATION THERMIQUE α-MANNOSIDASE/PHOSPHATASE ACIDE
#### (en %)

| CF-hétérozygotes | 0' | 40' | 120' | 200' |
|---|---|---|---|---|
| 19 | 100/100 | 88/63 | 55/38 | 56/44 |
| 20 | 100/100 | 75/67 | 48/42 | 52/46 |
| 21 | 100/100 | 85/75 | 56/48 | 54/46 |
| 22 | 100/100 | 79/83 | 47/47 | 45/44 |
| moyenne | 100/100 | 75.4/69.3 | 52.5/45.6 | 50.7/44.7 |
| ± D.S. | | ±7.4/±8.0 | ±4.1/±5.1 | ±3.4/±5.2 |

### TABLEAU 1c

### INACTIVATION THERMIQUE α-MANNOSIDASE/PHOSPHATASE ACIDE
#### (en %)

| CF-homozygotes | 0' | 40' | 120' | 200' |
|---|---|---|---|---|
| 1 | 100/100 | 57/59 | 29/25 | 11/6 |
| 2 | 100/100 | 62/58 | 32/29 | 16/7 |
| 3 | 100/100 | 68/56 | 37/34 | 22/14 |
| 4 | 100/100 | 68/72 | 40/40 | 19/15 |
| 5 | 100/100 | 65/56 | 45/27 | 26/9 |
| moyenne | 100/100 | 64/60 | 36.6/31 | 18.8/10.2 |
| ± D.S. | | ±4.6/±6.7 | ±6.3/±6.0 | ±5.7/±4 |

### Revendications

1. Procédé de diagnostic *in vitro* de la fibrose kystique ou d'un défaut génétique transmissible, caractéristique de la fibrose kystique, caractérisé en ce que, partant d'un milieu biologique provenant soit directement de l'individu chez lequel est recherchée l'affection ou même la potentialité de transmettre l'affection, soit d'un milieu dans lequel des cellules préalablement prélevées sur cet individu avaient été préalablement cultivées ou mises en suspension, on détermine les conditions de température limites dans lesquelles au moins l'une des enzymes intervenant au niveau de la digestion intracellulaire, plus particulièrement des hydrolases, du type de celles qui sont susceptibles d'être affectées par le ou les défauts génétiques propres aux individus « CF-hétérozygotes » ou « CF-homozygotes » peut, lorsqu'elle est originaire d'un individu normal, rester stable, voire ne subir qu'une cinétique d'inactivation ralentie et, au contraire, subir une cinétique d'activation accélérée, voire une inactivation totale lorsqu'elle est originaire d'un individu « CF-hétérozygote » ou « CF-homozygote » et que l'on repère ensuite qualitativement ou quantitativement sur chacun des échantillons de milieu biologique à tester placés dans les mêmes conditions de température, les cinétiques d'inactivation accélérées ou altérations plus rapides éventuellement observables de la même enzyme, qui peuvent alors être corrélées au caractère « CF-hétérozygote » ou « CF-homozygote » des cellules correspondantes.

8

2. Procédé de diagnostic *in vitro* de la fibrose kystique ou d'un défaut génétique transmissible, caractéristique de la fibrose kystique, caractérisé en ce que, partant d'un milieu antérieurement en contact avec les cellules sur lesquelles doit être effectué ledit diagnostic, l'on place ledit milieu dans les conditions préalablement déterminées permettant à l'une au moins de celles des enzymes intervenant au niveau de la digestion intracellulaire qui ont été libérées dans le milieu et qui sont susceptibles de porter une modification lorsqu'elles sont originaires de cellules affectées par un défaut génétique corrélable à la fibrose kystique, de manifester une cinétique d'inactivation différentielle vis-à-vis de la cinétique d'inactivation de la même enzyme provenant de cellules saines dans les mêmes conditions prédéterminées de température, voire même une inactivation quasi totale, et l'on repère les variations éventuelles vis-à-vis de la normale de ladite cinétique d'inactivation, pour les corréler selon la variation mesurée à l'état, soit normal, soit « CF-hétérozygote », soit encore « CF-homozygote » des cellules dont l'enzyme était originaire.

3. Procédé selon la revendication 2, caractérisé en ce que la susdite détection consiste en la mesure, au bout d'un temps également prédéterminé, du degré d'inactivation éventuellement atteint, ce degré étant alors corrélable à l'un des états sus mentionnés desdites cellules.

4. Procédé selon l'une quelconque des revendications 1 à 3, caractérisé en ce qu'on opère au sein d'un milieu biologique à pH 5-9, de préférence sensiblement neutre.

5. Procédé selon la revendication 3 seule ou en combinaison avec la revendication 4, caractérisé en ce que l'enzyme choisie est constituée par l'α-mannosidase et que la susdite température limite est supérieure à 40 °C, notamment comprise entre 40 °C et 42 °C, de préférence de 41 °C ou 40,5 °C.

6. Procédé selon la revendication 3 seule ou en combinaison avec la revendication 4, caractérisée en ce que l'enzyme choisie est constituée par la phosphatase-acide et que la température recherchée est supérieure à 36 °C, notamment comprise entre 36 °C et 38 °C, de préférence de 36,5 °C.

7. Procédé selon l'une quelconque des revendications 1 à 8, caractérisé en ce que le milieu biologique est constitué par un plasma sanguin dont la fibrinogène ou les facteurs susceptibles d'intervenir de façon essentielle dans la coagulation ont été retirés ou par un plasma dans lequel ces facteurs sont totalement inhibés, notamment par une teneur de ce plasma en un anticoagulant, tel que l'héparine.

8. Procédé selon l'une quelconque des revendications 1 à 6, caractérisé en ce que le milieu biologique est constitué par un autre liquide biologique d'origine humaine, tel que l'urine ou le liquide amniotique prélevé chez la femme enceinte.

9. Procédé selon l'une quelconque des revendications 1 à 6, caractérisé en ce que le susdit milieu est constitué par un milieu de culture classique approprié à la culture de fibroblastes humains.

10. Procédé selon l'une quelconque des revendications 1 à 9, caractérisé en ce que les repérages, qualitatifs ou quantitatifs, sont effectués sur des échantillons du susdit milieu ayant un volume compris entre 0,1 et 100 microlitres, à l'abri de l'évaporation, notamment au moyen d'une couche d'huile de densité inférieure à 1, n'ayant qu'une tension de vapeur extrêmement réduite, sinon pratiquement nulle, à la température à laquelle sont effectués les essais d'inactivation, cette couche d'huile recouvrant l'échantillon traité, lorsque le test est effectué en tube.

11. Procédé selon la combinaison des revendications 3, 5 et 7, caractérisé en ce que ledit plasma sanguin est maintenu à 41 °C pendant une durée de 200 minutes et que l'on mesure le pourcentage d'inactivation de l'α-mannosidase obtenue après ce traitement thermique, les cellules dont provenait l'α-mannosidase étudiée étant alors considérées comme vraisemblablement « CF-homozygotes » ou « CF-hétérozygotes », selon que le pourcentage d'inactivation mesuré est contenu dans l'un des intervalles respectifs suivants : 75-80 % ou 40-60 %, un pourcentage d'inactivation contenu dans un intervalle 25-35 % témoignant d'une vraisemblance, sinon d'une certitude du caractère hétérozygote des cellules correspondantes.

## Claims

1. Process for the diagnosis *in vitro* of cystic fibrosis or of a transmissible genetic defect characteristic of cystic fibrosis, characterized in that, starting from a biological medium originating either directly from the individual in whom the disease is to be detected or even the potentiality of transmitting the disease, or from a medium in which cells previously taken from that individual were previously cultivated or suspended, one determines the limit temperature conditions under which at least one of the enzymes taking part at the level of the intracellular digestion, more particularly hydrolases of the type of those which are liable of being affected by the or any genetic defects specific to « CF-heterozygotes » or «CF-homozygotes » individuals, may, when it originates from a normal individual, remain stable or at most only undergo reduced inactivation kinetics and, to the contrary, undergo accelerated activation kinetics or even total inactivation when it originates from a « CF-heterozygote » or « CF-homozygote » individual and that one detects thereafter qualitatively or quantitatively on each of the samples of the biological medium to be tested brought into the same temperature conditions, the accelerated inactivation kinetics or more rapid alterations possibly observed of the same enzyme, which may then be correlated to the « CF-heterozygote » or « CF-homozygote » character of the corresponding cells.

2. Process of diagnostic *in vitro* of cystic fibrosis or of transmissible genetic defect characteristic of cystic fibrosis characterized in that, starting from a medium previously in contact with the cells on which said diagnosis is to be carried out, the said medium is placed under conditions previously determined enabling at least one of the enzymes taking part at the level of intracellular digestion and which were released in the medium and which are liable of carrying a modification when they originate from cells affected by a genetic defect correlatable to cystic fibrosis, of manifesting differential inactivation kinetics with respect to the inactivation kinetics of the same enzyme originating from healthy cells under the same predetermined conditions of temperature, or even a quasi-total inactivation, and that the possible variations of said inactivation kinetics as compared to normal, are detected for the sake of correlating them according as the measured variations, to either the normal or « CF-heterozygote » state or to the « CF-homozygote » state of the cells from which the enzyme originated.

3. Process according to claim 2 characterized in that the above detection consists in the measurement after a time which had been predetermined too, of the inactivation degree possibly reached, this degree being then correlatable to one of the above indicated states of said cells.

4. Process according to anyone of claims 1 to 3, characterized in that one operates within a biological medium at pH 5-9, preferably substantially neutral.

5. Process according to claim 3 alone or in combination with claim 4, characterized in that the enzyme selected is formed by $\alpha$-mannosidase and that the abovesaid limit temperature is higher than 40 °C, particularly comprised between 40 °C and 42 °C, preferably of 41 °C or 40.5 °C.

6. Process according to claim 3 alone or in combination with claim 4, characterized in that the enzyme selected is formed of acid phosphatase and that the temperature considered is higher than 36 °C, particularly comprised between 36 °C and 38 °C, preferably of 36.5 °C.

7. Process according to anyone of claims 1 to 8, characterized in that the biological medium is constituted by a blood plasma whose fibrinogen or factors liable of taking part in an essential manner in the coagulation has been removed or by a plasma in which said factors were totally inhibited, particularly by means of a content in said plasma of an anticoagulant, such as heparin.

8. Process according to anyone of claims 1 to 6, characterized in that the biological medium is formed by another biological liquid of human origin, such as urine or amniotic liquid taken up from pregnant woman.

9. Process according to anyone of claims 1 to 6, characterized in that said medium is formed of a conventional culture medium suitable for the cultivation of human fibroblasts.

10. Process according to anyone of claims 1 to 9, characterized in that the qualitative or quantitative detections are carried out on samples of said medium having a volume comprised between 0.1 and 100 microlitres shielded from evaporation, particularly by means of an oil layer having a density lower than 1, having but an extremely reduced vapor tension, when not practically nil, at the temperature at which the inactivation assays are carried out, this oil layer lying over the treated sample, when the test is carried out in tube.

11. Process according to the combination of claims 3, 5 and 7, characterized in that said blood plasma is maintained at 41 °C for a period of 200 minutes and that one measures the inactivation percentage of $\alpha$-mannosidase obtained after said thermal treatment, the cells from which the studied $\alpha$-mannosidase was originating then being considered as likely being « CF-homozygote » or « CF-heterozygote » according as the inactivation percentage measured is comprised within one of the following respective intervals : 75-80 % or 40-60 %, a percentage of inactivation comprised within a 25-35 % interval witnessing a likelyhood, if not a certitude, of the heterozygote character of the corresponding cells.

**Ansprüche**

1. Verfahren zur in vitro-Diagnose der zystischen Fibrose oder eines erblichen genetischen Fehlers, der für die zystische Fibrose charakteristisch ist, dadurch gekennzeichnet, daß man ausgehend von einem biologischen Medium, das entweder direkt von dem Individuum, bei dem die Erkrankung oder die Möglichkeit der Vererbung der Erkrankung festgestellt werden soll, oder von einem Medium stammt, in dem Zellen, die diesem Individuum zuvor entnommen worden sind, zuvor gezüchtet oder suspendiert worden sind, die Temperaturgrenzbedingungen bestimmt, bei denen mindestens eines der Enzyme, die im Bereich der intrazellulären Verdauung eingreifen, insbesondere Hydrolasen, des Typs von Zellen, die durch den oder die genetischen Fehler, der bzw. die den « ZF-heterozygoten » oder « ZF-homozygoten » Individuen eigen ist bzw. sind, beeinflußt werden, dann stabil bleibt, d. h. nur einer verlangsamten Inaktivierungskinetik unterliegt, wenn es von einem normalen Individuum stammt, und andererseits einer beschleunigten Inaktivierungskinetik, d. h. einer vollständigen Inaktivierung unterliegt, wenn es von einem « ZF-heterozygoten » oder « ZF-homozygoten » Individuum stammt ; und anschließend für sämtliche zu testenden Proben des biologischen Mediums, die unter gleichen Temperaturbedingungen gehalten werden, qualitativ oder quantitativ die beschleunigten Inaktivierungskinetiken oder schnelleren Veränderungen, die gegebenenfalls für das gleiche Enzym beobachtet werden können, feststellt, die dann mit dem « ZF-heterozygoten » oder « ZF-homozygoten » Charakter der entsprechenden Zellen in Korrelation gebracht werden können.

2. Verfahren zur in vitro-Diagnose der zystischen Fibrose oder eines erblichen genetischen Fehlers, der für die zystische Fibrose charakteristisch ist, dadurch gekennzeichnet, daß man ausgehend von einem Medium, welches zuvor mit den Zellen in Kontakt gebracht worden ist, an denen die Diagnose durchgeführt werden soll, dieses Medium zuvor ermittelten Bedingungen unterwirft, bei denen mindestens eines jener Enzyme, die im Bereich der intrazellulären Verdauung eingreifen, die in dem Medium freigesetzt worden sind und die einer Veränderung unterliegen, wenn sie von Zellen stammen, die von einem genetischen Fehler, der mit der zystischen Fibrose in Korrelation gebracht werden kann, beeinflußt sind, indem sie gegenüber der Inaktivierungskinetik des gleichen, von gesunden Zellen stammenden Enzyms bei den gleichen vorbestimmten Temperaturbedingungen eine andersartige Inaktivierungskinetik, d. h. sogar eine quasi vollständige Inaktivierung zeigen ; und die gegebenenfalls vorhandenen Änderung gegenüber der normalen Inaktivierungskinetik feststellt, um über die gemessene Änderung eine Korrelation mit dem entweder normalen oder dem « ZF-heterozygoten » oder dem « ZF-homozygoten » Zustand der Zellen, aus denen das Enzym stammte, zu erreichen.

3. Verfahren nach Anspruch 2, dadurch gekennzeichnet, daß der Nachweis darin besteht, daß man nach dem Ablauf einer ebenfalls vorbestimmten Zeitdauer den gegebenenfalls erreichten Inaktivierungsgrad mißt, der mit einem der oben erwähnten Zustände der genannten Zellen in Korrelation gebracht werden kann.

4. Verfahren nach einem der Ansprüche 1 bis 3, dadurch gekennzeichnet, daß man in einem biologischen Medium mit einem pH-Wert von 5 bis 9 und vorzugsweise einem im wesentlichen neutralen Medium arbeitet.

5. Verfahren nach Anspruch 3 allein oder in Kombination mit Anspruch 4, dadurch gekennzeichnet, daß das ausgewählte Enzym α-Mannosidase ist und daß die Grenztemperatur oberhalb 40 °C, insbesondere zwischen 40 °C und 42 °C liegt und noch bevorzugter 41 °C oder 40,5 °C beträgt.

6. Verfahren nach Anspruch 3 allein oder in Kombination mit Anspruch 4, dadurch gekennzeichnet, daß das ausgewählte Enzym Saure Phosphatase ist und die gewünschte Temperatur oberhalb 36 °C und insbesondere zwischen 36 und 38 °C und vorzugsweise bei 36,5 °C liegt.

7. Verfahren nach einem der Ansprüche 1 bis 6, dadurch gekennzeichnet, daß das biologische Medium ein Blutplasma, dem das Fibrinogen oder Faktoren, die im wesentlichen Umfang in die Koagulation eingreifen, entzogen worden sind, oder ein Plasma ist, in dem diese Faktoren vollständig inhibiert sind, insbesondere dadurch, daß das Plasma ein Antikoagulans, wie Heparin, enthält.

8. Verfahren nach einem der Ansprüche 1 bis 6, dadurch gekennzeichnet, daß das biologische Medium eine andere biologische Flüssigkeit menschlichen Ursprungs ist, wie Urin oder schwangeren Frauen entnommenes Fruchtwasser.

9. Verfahren nach einem der Ansprüche 1 bis 6, dadurch gekennzeichnet, daß das Medium ein klassisches Kulturmedium ist, das zur Züchtung von Humanfibroblasten geeignet ist.

10. Verfahren nach einem der Ansprüche 1 bis 9, dadurch gekennzeichnet, daß die qualitativen oder quantitativen Bestimmungen an Proben des Mediums durchgeführt werden, die ein Volumen zwischen 0,1 und 100 µl aufweisen und bei denen das Verdampfen verhindert wird, insbesondere mit Hilfe einer Schicht eines Öls mit einer Dichte von weniger als 1, welches einen extrem niedrigen, wenn nicht praktisch keinen Dampfdruck bei der Temperatur aufweist, bei der die Inaktivierungsuntersuchungen durchgeführt werden, welche Ölschicht die behandelte Probe bedeckt, wenn der Test in einem Röhrchen durchgeführt wird.

11. Verfahren nach den Ansprüchen 3, 5 und 7, dadurch gekennzeichnet, daß das Blutplasma während einer Dauer von 200 Minuten bei 41 °C gehalten wird und man den Prozentsatz der Inaktivierung der α-Mannosidase nach dieser thermischen Behandlung mißt, wobei die Zellen, aus denen die untersuchte α-Mannosidase stammt, als wahrscheinlich « ZF-homozygot » oder « ZF-heterozygot » betrachtet wird in Abhängigkeit davon, ob der gemessene Inaktivierungsprozentsatz in einem der folgenden Intervalle liegt : 75 bis 80 % oder 40 bis 60 %, während ein Inaktivierungsprozentsatz im Bereich von 25 bis 35 % die Wahrscheinlichkeit, wenn nicht die Sicherheit eines heterozygoten Charakters der entsprechenden Zellen manifestiert.

Fig.1.

# Fig. 2.